Europäisches Patentamt

European Patent Office

Office européen des brevets

(19)

(11) Publication number: 0 338 593

A1

# EUROPEAN PATENT APPLICATION

(12)

(21) Application number: 89107331.4

(22) Date of filing: 24.04.89

(51) Int. Cl.4: A61B 5/10 , A61C 19/04

(30) Priority: 22.04.88 JP 100885/88

(43) Date of publication of application:
25.10.89 Bulletin 89/43

(84) Designated Contracting States:
DE FR GB

(71) Applicant: DAIKIN INDUSTRIES, LIMITED
Umeda Center Building 4-12 Nakazaki-nishi
2-chome Kita-ku
Osaka 530(JP)

Applicant: Saito, Shigeru
8-17-40, Kuki
Zushi-shi Kanagawa-ken(JP)

(72) Inventor: Fukada, Eiichi
New Weruterasu C 406 1-9-27, Miyamaedaira
Miyamae-ku Kawasaki-shi Kanagawa-ken(JP)
Inventor: Saito, Shigeru
8-17-40, Kuki Zushi-shi
Kanagawa-ken(JP)
Inventor: Yagi, Toshiharu
8-3-26, Honmachi Toyonaka-shi
Osaka(JP)
Inventor: Higashihata, Yoshihide
Kotoku Haitsu 101 5-19-28, Senrioka-Higashi
Settsu-shi Osaka(JP)

(74) Representative: Patentanwälte Beetz sen. -
Beetz jun. Timpe - Siegfried -
Schmitt-Fumian
Steinsdorfstrasse 10
D-8000 München 22(DE)

(54) Mastication measurement apparatus and method.

(57) The mastication measurement apparatus of the invention is provided with a displacement detecting element such as a piezoelectric element and holding means which keeps the detecting element in close contact with a side part of a face, and is capable of processing the electric signals produced by the detecting element thereby to measure the number of masticating cycles, number of masticating cycles per unit time, a maximum value of the mastication force and the mastication energy, and also is capable of check to see whether a denture is matched to a subject by detecting noises included in the output of the detecting element. In accordance to the invention, it is also made possible to determine the teeth which are not used in mastication of food by attaching the detecting elements to both sides of a face and comparing the outputs from both detecting elements.

# MASTICATION MEASUREMENT APPARATUS AND METHOD

## BACKGROUND OF THE INVENTION

### 1. Field of the Invention

This invention relates to an apparatus for measuring mastication of humans and animals and method thereof.

### 2. Description of the Prior Art

With the rapid changes in the eating habits which increased tender foods to a considerable extent, mastication function of sufficient chewing and digestion is being lost. In children of lower grades of elementary schools, in particular, 75% of them do not or can not chew foods enough, according to a study. Such insufficient mastication results not only in physical disorders such as dysgnathia, teeth malalignment, dental caries and pyorrhea alveolaris, but also in mental disturbances such as hypomnesia.

For this reason, improvements of the entire eating habits and related environment is desired to encourage the public to masticate foods sufficiently.

On the other hand, as a transition of emphasis in medical practice from therapeutic medicine to preventive and control medicine is expected in the future, dentists will be required to give the patients post-therapeutic instructions and to control and prevent the recurrence, instead of ending in the therapeutic treatment. In this trend, research of mastication has been increasingly becoming important.

In a prior art of measuring the masticating cycles, picture of a subject recorded is with a video camera, and the mastication cycles and the factors are measured by playing back the recorded picture. Although this prior art is practical, it requires a video camera and related equipment and makes the test an elaborate procedure. The prior art also has a drawback that the measurement can not be made when the subject turns sideways instead of facing toward the camera.

Another prior art is the electromyograph method which includes a procedure similar to that of conventional electrocardiography, with sensors adhered to the face of the subject detecting the electric current thereby. These prior arts have the problems of being unable to measure the mastication easily.

## SUMMARY OF THE INVENTION

The object of the invention is to provide an apparatus and a method for measuring mastication simply and accurately.

The invention provides an apparatus for measuring mastication comprising:
an element for detecting a displacement a side part of a face, and
means for holding to keep the displacement detecting element in close contact with the side part of the face.

In a preferred embodiment of the invention, the displacement detecting element is a piezoelectric element.

In another preferred embodiment, the side part of the face is a part of a masseter.

In a different preferred embodiment, the holding means comprises a support member of which the overall configuration is U-shaped with both ends positioned at both sides of the face, and means for holding the displacement detecting element in a condition of facing the side part of the face which is fitted to at least one end of the holding member.

In a further preferred embodiment, the holding means is an adhesive tape for attaching the displacement detecting element on the side of the face.

In a still further preferred embodiment, the piezoelectric element is a bimorph element.

The invention provides an apparatus for measuring mastication comprising,
an element for detecting a displacement a side part of a face, and
means for holding to keep the displacement detecting element in close contact with the side part of the face, and
means for processing to operate in response to the output of the displacement detecting element.

In a preferred embodiment, the displacement detecting element is a piezoelectric element.

In a preferred embodiment, the processing means counts a number of masticating cycles.

In another preferred embodiment, the processing means counts the number of masticating cycles per unit time.

In a different preferred embodiment, the processing means measures a mastication force corresponding to a peak value of the output of the displacement detecting element.

In a further preferred embodiment, the processing means integrates the output of the displacement detecting element and measures a mastication energy corresponding to the integral value.

2

In a still preferred embodiment, the processing means detects noises included in the output of the displacement detecting element.

In a preferred embodiment, the processing means is capable of changing the threshold for the output of the displacement detecting element and counts the number of masticating cycles corresponding to the output.

The invention relates to a method for measuring mastication comprising steps of;
attaching a displacement detecting element to a side part of a face, and
displaying an output of the displacement detecting element by display means.

In accordance to the invention, because the displacement detecting element, for example a piezoelectric sensor, strain gage or the like, is held in close contact with the side part of a face by the holding means, mastication of a human or an animal can be measured in a natural condition, and the measurement is accurate and simple.

In accordance to the invention, the output of the displacement detecting element is processed by the processing means, thereby the number of mastication cycles, the number of mastication cycles per unit time, the mastication force and the mastication energy can be measured. Moreover, the noises included in the output of the displacement detecting element is detected to check, in case the subject has a false tooth or false teeth, to see whether it or they are well fitted.

As is evident from the preceding description, because the invention enables to measure the mastication by the displacement detecting element such as piezopelectric element, the mastication measurement can be performed simply and accurately, and therefore no particular trouble is required of the subject other than masticating naturally, and fitting of the measurement apparatus is comfortable.

BRIEF DESCRIPTION OF THE DRAWINGS

The object previously described, other objects, features and advantages of the invention will be better understood when taking in conjunction with the following detailed description and the drawings.

Fig.1 is a view showing an entire system of an embodiment of the invention.

Fig.2 is a cross sectional view of a part of the apparatus shown in Fig.1.

Fig.3 is a front view of the holding means 7 with a cover 12 removed;

Fig.4 is a view showing waveforms of outputs from the displacement detecting element 3.

Fig.5 is a cross sectional view of a part of another embodiment of the invention.

Fig.6 is a front view of the embodiment shown in Fig.5. Detailed description of a preferred embodiment of the invention.

DETAILED DESCRIPTION OF THE PREFERRED EMBODIMENTS

A preferred embodiment of the invention is explained in the following referring to the drawings.

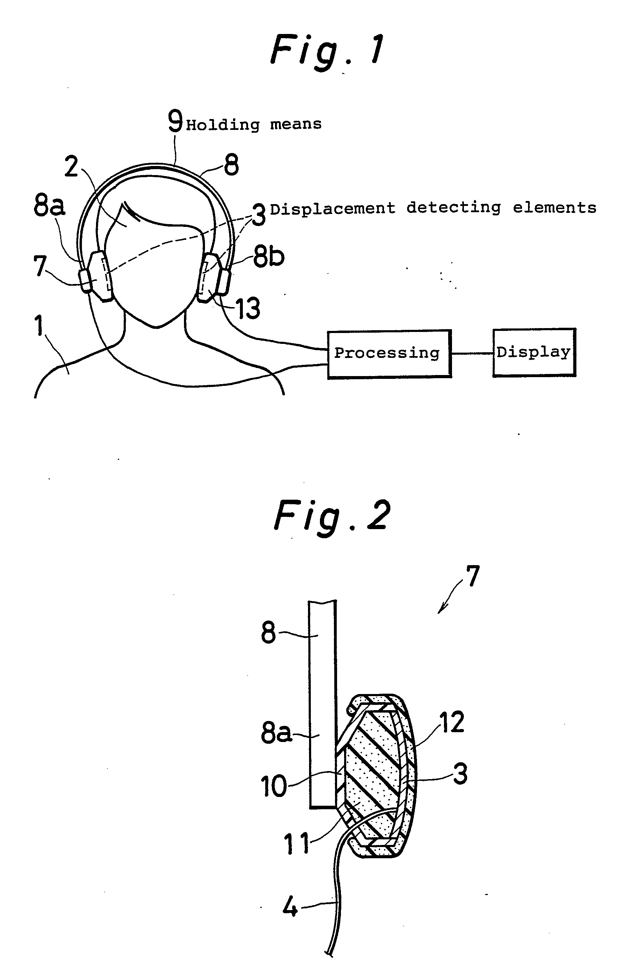

Fig.1 shows the entire system of an embodiment of the invention and Fig.2 is a cross sectional view thereof. A displacement detecting element 3 is attached on a side part of a face 2 subject 1 whose mastication is to be measured and the output from the displacement detecting element 3 is fed via a lead wire 4 to signal processing means 5 which incorporates a micro computer and other equipment. Measurement result obtained by the processing means 5 is displayed by display means 6.

The side part of the face 2 refers to a part of the face which makes displacement during mastication, or more specifically, a part on the masseter or temporal muscle. Because a part which shows displacements of the largest magnitude and displacement during mastication among these parts is the masseter, this part is preferably used. Particularly because the displacement is detected only during mastication and displacements of parts of the face and check during conversation, for example, are not detected in case the displacement detecting element is pressed against the masseter, this part is most suitable for use in the measurement of the number of mastication cycles and mastication force.

The displacement detecting element 3 is fitted to a holding member 8 by fitting means 7 while the holding member 8 and the fitting means 7 constitute holding means 9. The holding member 8 is an elastic U-shaped band made of a metal, for example. One end 8a of the holding member 8 is fitted with the fitting means 7. The fitting means 7 is provided with a housing 10 and an elastic body 11 filling the housing 10 and the displacement detecting element 3 is held on the elastic body 11. The elastic body 11 is made of a material such as, a foamed synthetic resin or rubber. The displacement detecting element 3 is flat and convex toward the side part of the face 2, and is elastically pressed against the face 2 by the elastic body 11. Periphery of the displacement detecting element 3 and a part of the housing 10 are covered with a cover 12. The cover 12, made of a thin sponge or cloth, makes the feeling of the contact to the side part of the face 2 comfortable as well as transmit the displacement of the side part of the face 2 to the displacement detecting element 3 certainly.

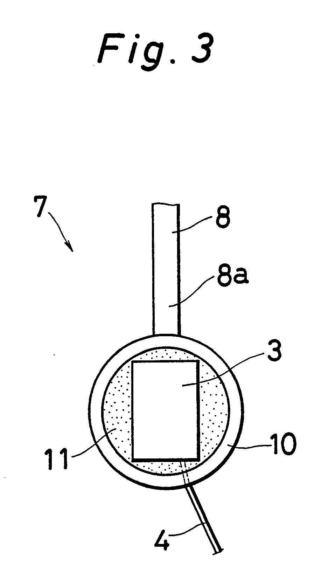

Fig.3 is a front view of the fitting means 7 shown in Fig.2 with the cover 12 removed. The front face of the displacement detecting element 3 is made in, for example, a rectangular shape.

The displacement detecting element 3 may be a pressure sensitive element such as a piezoelectric element or a strain gage, or any other element which detects the displacement of the side part of the face 2, and generates an electric signal of a level corresponding to the displacement of the side part of the face 2 during mastication. As the piezoelectric element, (a) high-polymer piezoelectric material, (b) ceramics piezoelectric material or (c) compound of a high-polymer material and a ceramics piezoelectric material. (a) high-polymer piezoelectric material may be a natural high-polymer piezoelectric material or a synthetic high-polymer piezoelectric material, where the synthetic high-polymer piezoelectric material may be polyvinylidene fluoride, vinylidene fluoride copolymer or copolymer of vinylidene cyanide and vinyl acetate. (b) ceramics piezoelectric material may be barium titanium ($BaTiO_3$) or lead zirconate titanate (PZT). (c) Compound piezoelectric material is obtained by mixing the above-mentioned ceramics material into the above-mentioned high-polymer material. Among these, in view of the flexibility, resistance to breakage and conformability to the displacement of a part of the face during mastication, the piezoelectric element using high-polymer material of (a) or (c) is preferred for the object of the invention.

The shape of the piezoelectric element may be so-called single structure, bimorph structure or multiple-layer structure, but in order to detect the change in the survature of the part of the face due to the projection of the masseter during mastication such as described previously, the piezoelectric element of bimorph structure is preferred.

While the displacement detecting element 3 is attached to at least one end 8a of the holding member 8, usually it can be attached also to another end 8b similarly by the fitting means 13.

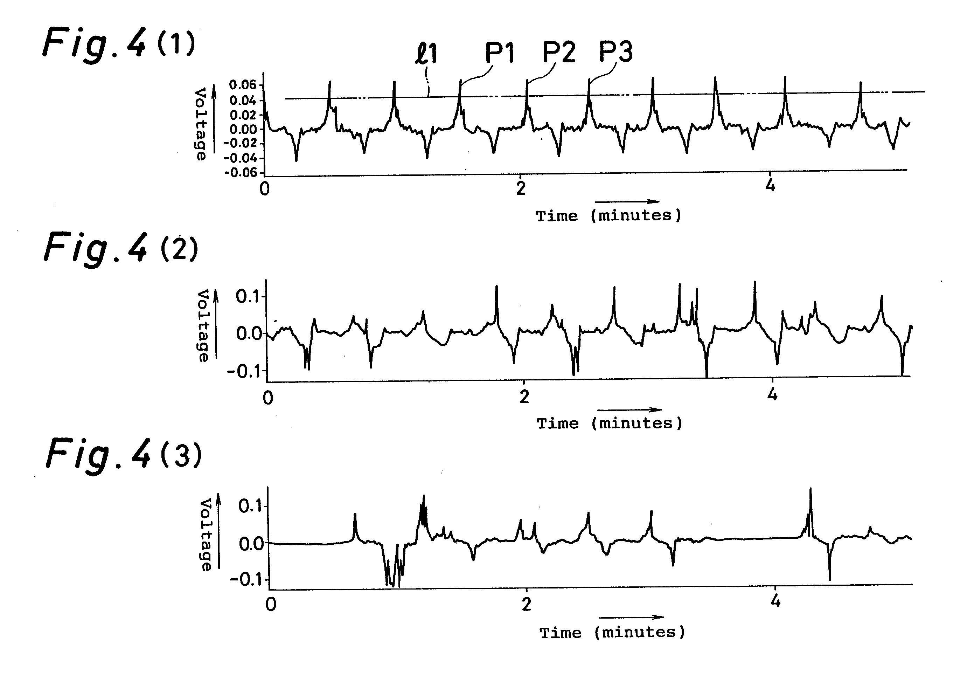

Fig.4 shows a waveform of the output from the displacement detecting element 3 when it is a piezoelectric element. Fig.4 (1) shows a waveform obtained when the subject 1 repeats chewing movement without holding nothing in the mouth, Fig.4 (2) is a part of waveform obtained when a male subject chews a gum and Fig.4 (3) is a part of waveform obtained when a female subject chews a gum. As shown in Fig. 4 (1), for example, number of masticating cycles of the subject 1 is counted by the processing means 5 by discriminating the pulses obtained from the displacement detecting element 3 with reference to a predetermined threshold ℓ1 and counting the pulses p1, p2, p3 which have levels higher than the threshold ℓ1.

By counting the number of pulses p1, p2, p3 per unit time, number of masticating cycles per unit time, namely the mastication speed can be measured.

The peak value of the output pulses p1, p2, p3 of the displacement detecting element 3 corresponds to the mastication force and therefore the strength difference of the mastication force by the kind of food, tender or hard, and the individual difference can be measured.

The processing means 5 is made to count the pulses p1, p2, p3 which have levels higher than the threshold ℓ1, and therefore to count only the mastication cycles of mastication forces greater than that corresponding to the threshold ℓ1, thereby enabling to give instructions to the subject 1 such as a child to improve the mastication force.

The processing means can also integral the pulses p1, p2, p3 individually and thereby measure the mastication energy, thus making it possible to analyse the mastication energy consumed when eating various foods.

Further the processing means 5 is capable of detecting noises included in the pulses p1, p2, p3 and thereby checking to see, when applying a denture to the subject 1, whether the denture is matched to the subject 1. In case the denture is not matched to the subject 1, much noise is detected when the subject 1 chews the gum or the like. Thus, while matching of the denture has conventionally been checked by dentists testing for a long time, for example 2 or 3 days, in accordance to the invention, matching of the denture can be tested in a very short time with the denture fitted to the subject 1.

Further, use of a side which includes an impairment, a weak side as it were, among left and right sides of the teeth is generally avoided during mastication. Therefore this invention can be applied also to the detection of the weak side of the teeth earlier. For such diagnosis, output waveforms of the displacement detecting elements 3 attached by the right and left fitting means 7 and 13 are compared. The output waveform obtained from the one of the two displacement detecting elements attached to the side of weak teeth which is not used in mastication has lower amplitude. Therefore it can be judged that the teeth on the side of the displacement detecting element 3 which gives smaller output are the weaker teeth.

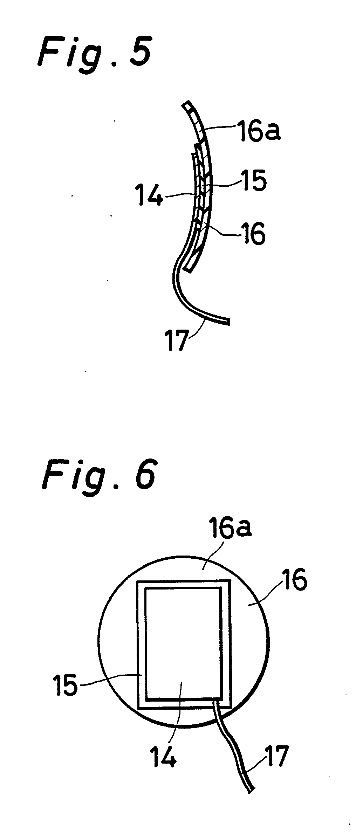

Fig.5 is a cross sectional view of a part of another embodiment of the invention and Fig.6 is a front view thereof. A displacement detecting element 14 is attached to a part of an adhesive tape 16 via an elastic body 15. In this case, the elastic body 15 is not necessary. Mastication can be measured by attaching a part 16a of the adhesive tape 16 exposed out of the displacement detecting element 14 and the elastic body 15 to a side part of the face 2 of a subject 1. A flexible lead wire 17 is connected to the displacement detecting element 14. Other constitution is the same as the embodiment previously described.

In a case where a ceramics piezoelectric element is used as the displacement detecting element 3, when the ceramics piezoelectric element made in a rigidly constructed, the elastic body 11 is substituted with a rigid member in order to enable to detect the piezoelectricity in the thickness direction of the ceramics piezoelectric element.

In accordance to the invention, mastication can be measured by observing waveform displayed on an oscilloscope or the like by attaching the displacement detecting element to the side part of the face and having the output of the displacement detecting element on the oscilloscope or the like.

The invention may be embodied in other specific forms without departing from the spirit or essential characteristics thereof. The present embodiments are therefore to be considered in all respects as illustrative and not restrictive, the scope of the invention being indicated by the appended claims rather than by the foregoing description and all changes which come within the meaning and the range of equivalency of the claims are therefore intended to be embraced therein.

Claims

1. An apparatus for measuring mastication comprising:
an element for detecting a displacement a side part of a face, and
means for holding to keep the displacement detecting element in close contact with the side part of the face.

2. An apparatus for measuring mastication as claimed in claim 1, wherein the displacement detecting element is a piezoelectric element.

3. An apparatus for measuring mastication as claimed in claim 1, wherein the side part of the face is a part of a masseter.

4. An apparatus for measuring mastication as claimed in claim 1, wherein the holding means comprises a support member of which the overall configuration is U-shaped with both ends positioned at both sides of the face, and means for holding the displacement detecting element in a condition of facing the side part of the face which is fitted to at least one end of the holding member.

5. An apparatus for measuring mastication as claimed in claim 1, wherein the holding means is an adhesive tape for attaching the displacement detecting element on the side of the face.

6. An apparatus for measuring mastication as claimed in claim 2, wherein the piezoelectric element is a bimorph element.

7. An apparatus for measuring mastication comprising,
an element for detecting a displacement a side part of a face, and
means for holding to keep the displacement detecting element in close contact with the side part of the face, and
means for processing to operate in response to the output of the displacement detecting element.

8. An apparatus for measuring mastication as claimed in claim 7, wherein the displacement detecting element is a piezoelectric element.

9. An apparatus for measuring mastication as claimed in claim 7, wherein the processing means counts a number of masticating cycles.

10. An apparatus for measuring mastication as claimed in claim 7, wherein the processing means counts the number of masticating cycles per unit time.

11. An apparatus for measuring mastication as claimed in claim 7, wherein the processing means measures a mastication force corresponding to a peak value of the output of the displacement detecting element.

12. An apparatus for measuring mastication as claimed in claim 7, wherein the processing means integrates the output of the displacement detecting element and measures a mastication energy corresponding to the integral value.

13. An apparatus for measuring mastication as claimed in claim 7, wherein the processing means detects noises included in the output of the displacement detecting element.

14. An apparatus for measuring mastication as claimed in one of claims 7-13, wherein the processing means is capable of changing the threshold for the output of the displacement detecting element and counts the number of masticating cycles corresponding to the output.

15. A method for measuring mastication comprising steps of;
attaching a displacement detecting element to a side part of a face, and
displaying an output of the displacement detecting element by display means.

# Fig. 1

9 Holding means

2

8

8a

8

3 Displacement detecting elements

8b

7

13

1

Processing

Display

# Fig. 2

7

8

8a

10

11

12

3

4

EP 0 338 593 A1

*Fig. 3*

EP 0 338 593 A1

*Fig.4* (1)

ℓ1   P1   P2   P3

Voltage

0.06
0.04
0.02
0.00
-0.02
-0.04
-0.06

0          2          4

Time (minutes)

*Fig.4* (2)

Voltage

0.1

0.0

-0.1

0          2          4

Time (minutes)

*Fig.4* (3)

Voltage

0.1

0.0

-0.1

0          2          4

Time (minutes)

# Fig. 5

# Fig. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-2 674 808 (G.W. TAINTER) * column 1, lines 14-26; lines 42-52; figure 1 * | 1,3,4 | A 61 B 5/10 A 61 C 19/04 |
| A | | 7,15 | |
| A | DE-A-3 248 179 (SIEMENS AG) * abstract; figure 1 * | 2,6,8 | |
| A | EP-A-0 108 341 (MYO-TRONICS RESEARCH, INC.) * abstract; figure 1 * | 1,12,13 | |
| A | MEDICAL & BIOLOGICAL. ENGINEERING & COMPUTING vol. 19, no. 6, November 1981, pages 789-791, November 1981, Stevenage, Herts, GB; H.F. ATKINSON et al.:"Technical note - Closed-circuit television movement detector" * page 789, left hand column, lines 1-10 * | 1,11 | |

TECHNICAL FIELDS SEARCHED (Int. Cl.4)

A 61 C 19/00
A 61 B 5/00

The present search report has been drawn up for all claims

| Place of search | Date of completion of the search | Examiner |
|---|---|---|
| BERLIN | 14-07-1989 | WEIHS J.A. |

EPO FORM 1503 03.82 (P0401)